Europäisches Patentamt

European Patent Office

Office européen des brevets

(19)

(11) Veröffentlichungsnummer: **0 214 330**
**B1**

(12) # EUROPÄISCHE PATENTSCHRIFT

(45) Veröffentlichungstag der Patentschrift:
**26.04.89**

(51) Int. Cl.⁴: **G01N 33/545**

(21) Anmeldenummer: **85111376.1**

(22) Anmeldetag: **09.09.85**

(54) **Verfahren zum Immobilisieren von Substanzen an Kunststoffestphasen und Kunststoffestphasen zur Verwendung in Immunosorbentassays.**

(43) Veröffentlichungstag der Anmeldung:
**18.03.87 Patentblatt 87/12**

(45) Bekanntmachung des Hinweises auf die Patenterteilung:
**26.04.89 Patentblatt 89/17**

(84) Benannte Vertragsstaaten:
**AT BE CH DE FR GB IT LI LU NL SE**

(56) Entgegenhaltungen:
**EP-A- 0 001 224**
**EP-A- 0 026 007**
**EP-A- 0 126 392**
**EP-A- 0 131 546**
**DE-A- 2 711 270**
**FR-A- 2 345 459**

(73) Patentinhaber: **PROGEN Biotechnik GmbH, Im Neuenheimer Feld 519, D-6900 Heidelberg(DE)**

(72) Erfinder: **Rauterberg, Dr. Ernst W, Roonstr, 2, 6900 Heidelberg(DE)**

(74) Vertreter: **Hach, Hans Karl, Dr., Tarunstrasse 23, D-6950 Mosbach-Waldstadt(DE)**

## Beschreibung

Die vorliegende Erfindung bezieht sich auf ein Verfahren zum Immobilisieren von Substanzen an Kunststoffestphasen, die mit Weichmachern versetzt sind.

In den letzten Jahren wurden zum Nachweis einer großen Anzahl verschiedenster Substanzen Verfahren entwickelt, die auf immunologischen Reaktionsweisen beruhen und als Immunosorbentassays bezeichnet werden. Grundsätzlich beruhen diese Assays auf Antikörper-Antigen-Bindungen, die in der einen oder anderen Weise abgewandelt und verfeinert sein können. Beispielsweise kann das Erkennbarmachen der Antikörper-Antigen-Reaktion durch eine Enzymreaktion erfolgen oder durch die Markierung mit Radioisotopen. Im ersteren Fall handelt es sich dann um die durch Engvall und Perlmann (Immunochemistry 8:871) eingeführten enzymabhängigen Immunosorbentassays (ELISA). Diese Verfahren sind inzwischen weitgehend verbreitet und nehmen neben den radioimmunologischen Methoden (RIA) mehr und mehr Raum ein. Beide Verfahren sind heute fast überwiegend Assays, die mit festen Phasen arbeiten, wobei entweder zum Nachweis einer Substanz ein Molekül mit spezifischer Bindungseigenschaft gegen diese Substanz, beispielsweise ein Antikörpermolekül, Protein A oder ein Rezeptor an eine feste Phase gebunden wird oder umgekehrt zum Nachweis der genannten Moleküle die an diese Moleküle bindende Substanz primär an die Festphase gebunden wird. Die auf diese Weise beschichtete Festphase wird dann mit der Lösung inkubiert, die diejenige Substanz enthält, die zu messen ist, wobei es sich dabei, je nach dem, womit die Festphase beschichtet wurde, entweder um die genannte, zu messende Substanz oder die oben beispielsweise genannten Antikörpermoleküle, Protein A oder einen Rezeptor handelt. Im Falle des ELISA's wird sodann durch eine dritte Inkubation die zu messende Substanz mittels enzymgekoppelten Antikörpern entdeckt und sodann die Menge dieser enzymgekoppelten, gebundenen Sekundärantikörper durch eine Enzymreaktion meßbar gemacht. Es wurden nun noch eine Reihe von Variationen zu diesem Typ des ELISA's entwickelt, beispielsweise kompetitive ELISA's und ähnliches; sämtliche Immunosorbentassays beruhen jedoch darauf, daß ein erster Reaktant an eine feste Phase gebunden wird.

Überwiegend handelt es sich bei der Bindung des ersten Reaktanten an die feste Phase um eine hydrophobe Bindung. Die heute sowohl im wissenschaftlichen als auch kommerziellen Bereich in erster Linie verwendeten Festphasen bestehen aus hydrophoben Kunststoffen, wie etwa Polystyrol (PS) oder Polyvinylchlorid (PVC). Die ELISA-Technik beispielsweise hat nun insbesondere durch die Einführung von sogenannten Mikrotiterplatten und für diese besonders konstruierten Photometern, mit denen beispielsweise durch Enzymreaktionen hervorgerufene Farbveränderungen gemessen werden können, großen Auftrieb erhalten. Die heute auf dem Markt befindlichen Mikrotiterplatten bestehen nahezu alle aus PS oder PVC.

Die hydrophobe Adsorption von ersten Reaktanten ist jedoch mit gewissen Nachteilen behaftet.

Beispielsweise können nur Reaktanten gebunden werden, die selbst hydrophobe Areale auf ihrer Moleküloberfläche aufweisen. Diese müssen eine gewisse, bisher nicht genau zu bestimmende Größe haben. Auf diese Weise ist es sehr schwierig, stark geladene Reaktanten, beispielsweise Doppelstrang-DNS oder sehr kleine Substanzen, wie etwa Somatostatin, adsorptiv an PS- oder PVC-Mikrotiterplatten zu binden.

Während der zahlreichen Inkubations- und Waschschritte bei Immunosorbentassays, wie sie oben beschrieben wurden, wird ein von den Eigenschaften des jeweiligen Reaktanten abhängiger Anteil aus seiner Adsorptivbindung gelöst. Infolge davon verringert sich die Empfindlichkeit der Testsysteme und die Reproduzierbarkeit nimmt ab. Der Zusatz von Detergenzien bei den zahlreichen Wasch- und Inkubationsschritten verstärkt den genannten negativen Effekt, da gerade der Zusatz der Detergenzien weitere, adsorptive hydrophobe Bindungen der Reaktionspartner der ersten Reaktanten an die Festphase unterdrücken soll.

Zur Verbesserung der Bindung der ersten Reaktanten an die Festphase wurden alternativ zu der hydrophoben adsorptiven Festphasenbindung in der Vergangenheit ELISA-Systeme mit kovalenter Kupplung der ersten Reaktanten an die Festphase beschrieben. (Römer und Rauterberg, Immunobiol., vol. 166:24 (1984)). Mit dieser kovalenten Bindung, beispielsweise an Glas als Festphase, ließ sich eine wesentlich festere und dichtere Beschichtung der Oberfläche mit den ersten Reaktanten erreichen.

Die Herstellung von Mikrotiterplatten, mit denen Immunosorbentassays nahezu ausschließlich durchgeführt werden, da mit diesen Mikrotiterplatten mit wünschenswert kleinen Volumina gearbeitet werden kann, ist jedoch bezüglich der Festphase Glas, an die die wünschenswerte kovalente Bindung der ersten Reaktanten erfolgen könnte, äußerst aufwendig und kostenintensiv. Es hat daher im Stand der Technik nicht an Versuchen gefehlt, die Kunststoffe, aus denen Mikrotiterplatten verhältnismäßig einfach und billig hergestellt werden können, an ihrer Oberfläche so zu modifizieren, daß entweder aktivierbare Gruppen, beispielsweise primäre Aminogruppen, eingeführt wurden, oder die Mikrotiterplatten an ihrer Oberfläche beispielsweise mit einer lackähnlichen Schicht überzogen wurden, die ebenfalls aktivierbare Gruppen enthielt. Das letztgenannte Verfahren scheiterte daran, daß die verwendeten Lacke nur in stark hydrophoben Lösungsmitteln löslich waren, die ihrerseits die Kunststoffmikrotiterplatten auflösten. PS- und PVC-Mikrotiterplatten scheinen per se inert und für eine Einführung reaktiver Gruppen ungeeignet zu sein. Eine Einführung von Nitrogruppen in PS, die später zu Aminogruppen reduziert werden sollten, führte beispielsweise zur Zerstörung der Platten. Es ist jedoch nach wie vor sinnvoll, an dem Konzept der Mikrotiterplatten festzuhalten, da bei diesen Meßverfahren nur geringe Probenvolumina benötigt werden, wodurch die Kosten für sämtliche zu verwendenden Reagenzien niedriger gehalten werden können, als beispielsweise bei Einzelröhrchen-Messungen. Darüber hinaus ist eine Automatisierung möglich.

Die heute kommerziell vertriebenen Mikrotiterplatten werden üblicherweise während ihrer Herstellung mit Additiven oder Weichmachern behandelt. Dabei werden beispielsweise dem Kunststoffrohmaterial Mineralöl und Stearate oder Phthalsäuresalze in ppm-Mengen zugesetzt.

Es war Aufgabe der Erfindung, an die mit den Additiven oder Weichmachern behandelten Kunststoffestphasen eine qualitativ und quantitativ verbesserte Bindung der ersten Reaktanten an die Kunststoffestphase zu erreichen.

Diese Aufgabe wird dadurch gelöst, daß geeignete aktivierbare Gruppen in den Weichmachern oder Additiven verwendet werden, um die Substanzen an die Kunststoffestphasen zu binden oder zu diesem Zweck aktiviert oder mit bi- oder multivalenten Reaktanten umgesetzt werden.

Diese überraschende, erfindungsgemäße Lösung der oben formulierten Aufgabe resultierte aus Beobachtungen bei dem Versuch, DoppelstrangDNS direkt an PS-Platten zu binden. DoppelstrangDNS bindet, im Gegensatz zu Einzelstrang-DNS nicht spontan, also adsorptiv, an PS- oder PVC-Platten, so daß zusätzliche Maßnahmen ergriffen werden müssen, um die Doppelstrang-DNS an die Kunststoffestphase zu binden. Beispielsweise wurde versucht, Doppelstrang-DNS in Gegenwart von Carbodiimiden direkt an PS-Platten zu binden. Bei kinetischen Untersuchungen sowohl bei Raumtemperatur als auch bei 37°C wurde beobachtet, daß bei 37°C die Bindung von humanen Antikörpern gegen DNS nach 30 Minuten Inkubation deutlich größer war als bei 60 oder 120 Minuten. Diese Beobachtung war verblüffend, da eine solch vergleichsweise kurze Reaktionszeit nicht ausreicht, um alle Antikörper zu binden, d.h. also ein Gleichgewicht herzustellen.

Weiterhin konnte bei Raumpertur beobachtet werden, daß eine optimale Bindung bei einer Inkubationszeit von 120 Minuten erreicht wurde. Diese Beobachtung lag noch im Rahmen des Erwarteten. Wenn jedoch weiterhin, beispielsweise bis zu 240 Minuten inkubiert wurde, trat eine Vermindung der Bindung auf.

Dieser Effekt wäre erklärbar, wenn durch die Carbodiimide Gruppen von Substanzen aktiviert würden, die nicht fest in der Kunststoffphasenstruktur verankert sind, sondern durch längere Inkubation bzw. Inkubation bei höheren Temperaturen aus der Kunststoffestphase herauseluiert werden.

Es wurde nun festgestellt, daß es sich bei diesen, nicht fest in den Kunststoffphasen verankerten Substanzen um Weichmacher oder Additive handelt, mit denen die in den Labors verwendeten PSoder PVC-Mikrotiterplatten, die als Kunststoffestphase dienen, behandelt werden. Der beobachtete Effekt läßt sich so erklären, daß beispielsweise bei der Verwendung von Stearinen ein Teil der Carboxygruppen der Stearinsäuremoleküle bei den fertiggegossenen PS-Mikrotiterplatten für eine Aktivierung durch Carbodiimide erreichbar ist. Die Bindung der Stearinsäure ist aber wiederum nur hydrophob, wobei jedoch auf molekularer Ebene auch noch andere Mechanismen eine Rolle spielen können. Die hydrophobe Bindung jedoch könnte Ursache dafür sein, daß ein Teil der Stearinsäuremoleküle während einer verlängerten oder bei erhöhten Temperaturen durchgeführten Inkubation herauseluiert bzw. abgelöst wird.

Diese Hypothese läßt sich beispielsweise durch den Zusatz von Natrium-Stearat zu den Inkubationslösungen überprüfen. Durch die zusätzliche Zugabe von Natrium-Stearat sollte die Elution zumindest teilweise kompensiert werden. Ein Indiz für den Grad der Elution kann dabei beispielsweise der Grad der unspezifischen Bindung eines Serums sein. Unspezifische Bindung von Seren kommt höchstwahrscheinlich durch unbesetzte Stellen auf der Oberfläche der PS-Platten zustande. Durch die geschilderte Elution entstehen solche unbesetzten Stellen auf der Oberfläche der Kunststoffestphasen, so daß bei verstärker Elution auch eine Zunahme der unspezifischen Bindung eines Serums zu beobachten sein sollte. Diese Korrelation wurde durch die gezielte Zugabe von Natrium-Stearin gezeigt.

Die geschilderten Beobachtungen führten somit zu der vorliegenden Erfindung, die hydrophob an die Kunststoffestphasen adsorbierten Additive oder Weichmacher an ihren aktivierbaren Gruppen in der Weise chemisch zu aktivieren, daß Substanzen, die nicht im gewünschten Maße direkt an den Kunststoffestphasen gebunden werden können, über diese aktivierten Gruppen der Additive oder Weichmacher kovalent an die Kunststoffphase gebunden werden können.

Durch diese erfinderischen Maßnahmen läßt sich somit eine Bindungsqualität und -quantität erreichen, die bisher nur bei kovalenter Bindung von Substanzen an Oberflächen, wie beispielsweise Glas, beobachtet werden konnte.

Wie oben bereits dargelegt, erfolgt die Aktivierung der Additive oder Weichmacher idealerweise mit Carbodiimid-Derivaten.

Wenn die von den Additiven und Weichmachern zur Verfügung stehenden Gruppen für die Bindung der Reaktanten umgesetzt werden sollen, kann vorzugsweise Diglutaraldehyd verwendet werden.

Als Weichmacher oder Additive werden bevorzugt hydrophobe Substanzen mit aktivierbaren Gruppen verwendet, insbesondere Carboxygruppen, primäre Aminogruppen, –SH-Gruppen, Azidogruppen, Sulfosuccinimidylgruppen, Glyoxalgruppen, 2-Pyridyldithio-Gruppen, 4-Fluoro-3-Nitrophenylsulfon-Gruppen oder Isothiocyanogruppen.

Weiterhin erweisen sich als geeignete Substanzen für die hydrophobe Bindung an die Kunststofffestphase Imidioester, Phenazylbromide oder Phthalimide.

Besonders bevorzugt erscheint die Verwendung von Stearaten, vorzugsweise Zn- oder Na-Stearaten als Additive, da die Behandlung mit diesen Substanzen besonders kostengünstig ist.

Die ersten Reaktanten, die an die durch die aktivierten Additive oder Weichmacher vorbehandelten Kunststoffestphasen zu binden sind, sind vornehmlich Antikörper, Antigene, Haptene, Enzyme, DNS oder RNS. Wie bereits eingangs geschildert, sind die ersten Reaktanten jeweils ein Bestandteil einer Immunreaktion. Je nachdem, welche Substanz als erster Reaktant zur Bindung an die Kunststoffestphase dient, wird als Gegenreaktant die zu messende Substanz eingesetzt, bei der es sich dann entsprechend dem Charakter des ersten Reaktants wiederum jeweils um Antikörper, Antigene, Haptene, Enzyme, DNA oder RNS handeln kann. Wenn somit beispielsweise Antikörper gegen Doppelstrang-DNS fest und in optimaler Dichte an die durch Carbodiimide aktivierten Weichmacher und damit an die Kunststoffestphase immobilisiert werden, wird auf diese Weise ein Immuntestsystem zur Verfügung gestellt, das hochspezifisch Doppelstrang-DNS bindet. Es kann jedoch auch die Doppelstrang-DNS als erster Reaktant kovalent über die aktivierten Weichmacher oder Additive an die Kunststoffestphase gebunden werden. Sodann wird die derart beschichtete Kunststoffestphase mit dem Serum inkubiert, das die nachzuweisenden Antikörper gegen die Doppelstrang-DNS enthält.

Die vorzugsweise als Mikrotiterplatten ausgebildeten Kunststoffestphasen können aus den Kunststoffen Polystyrol oder Polyvinylchlorid bestehen, die sich als besonders geeignet erweisen.

Um das Herauseluieren der Additive und Weichmacher während der zahlreichen Inkubations- und Waschschritte noch möglichst weitgehend zu unterbinden, hat es sich als besonders vorteilhaft erwiesen, bei den Inkubationsschritten noch einmal zusätzlich diejenigen Weichmacher oder Additive zuzusetzen, mit denen die Kunststoffestphasen vorbehandelt wurden.

Die Erfindung bezieht sich auch auf Kunststoffestphasen, die mit Additiven oder Weichmachern behandelt wurden, die nach einem Verfahren gemäß einem der Ansprüche 1 bis 8 hergestellt wurden und in Immunosorbentassays verwendbar sind.

Als Immunosorbentassays eignen sich dabei sowohl enzymabhängige Immunosorbentassays (ELISA) als auch Radioimmunoassays (RIA). Wie bereits eingangs geschildert, unterscheiden sich die beiden genannten Assays in ihren Mitteln, mit denen die Antikörper-Antigen-Reaktion erkennbar gemacht werden kann. Aufgrund der gesteigerten Sensibilität der Bevölkerung gegenüber jederart von Radioaktivität haben sich in den letzten Jahren die enzymabhängigen Immunosorbentassays als die bevorzugten Testsysteme erwiesen.

Die erfindungsgemäßen Kunststoffestphasen stellen somit die Grundlage für eine quantitativ und qualitativ erheblich verbesserte Nachweismethode von Substanzen in Immuntestsystemen dar, wobei aufgrund der festeren und dichteren Bindung der ersten Reaktanten an die Kunststoffestphase auch die Reproduzierbarkeit der Ergebnisse erheblich erhöht werden konnte.

Die Erfindung wird nunmehr im folgenden Beispiel näher erläutert:

Beispiel 1

Es werden GREINER 96-Loch-Mikrotiterplatten oder DYNATECH Removawell Mikrotiterplatten eingesetzt. Diese Mikrotiterplatten bestehen aus PS, das mit Natriumstearat als Weichmacher versetzt ist. Diese Mikrotiterplatten werden mit 100 µl einer Lösung von 100 mg/ml 1-Cyclohexyl-3-(2-morpholino-ethyl)carbodiimid metho.4-toluolsufonat (Serva-HD, Nr. 17675) oder 1-Ethyl-3-(3-dimethylaminopropyl) carbodiimid-HCL (Serva-HD, Nr. 11250) oder N,N'-Dicyclohexylcarbodiimid pentachlorphenolkomplex (Serva-HD, Nr. 19491) in Aqua dest. (unmittelbar vor dem Eingeben gelöst) beschickt, um die Carboxygruppen der Stearinsäuremoleküle zu aktivieren, die zu immobilisierenden Substanzen über diese aktivierten Carboxygruppen kovalent an die Kunststoffphase der Mikrotiterplatte zu binden. Eine so vorbehandelte Mikrotiterplatte wird mit je 100 µl 5 µg/ml Kalbsthymus-DNS (Böhringer-Mannheim, Best. Nr. 104175) in Carbonat-Puffer beschickt. Nach einer Inkubation für 12 bis 16 Stunden bei 4–8°C werden die Lösungen entfernt und alle Vertiefungen jeweils mit 220 µl einer Lösung von 5 µl/ml Kalbsthymus-DNS gefüllt und für 3 Stunden bei Raumtemperatur unter Schütteln inkubiert. Nach dreimaligem Waschen mit je 300 µl 10 mM Phosphatpuffer, 150 mM NaCl, pH 7,2 (PBS), wird in die Löcher im Vierfachansatz jeweils 150 µl einer 1:100 Verdünnung von zu untersuchendem Humanserum (verdünnt mit PBS, das 0,5% Tween® 20 (Serva-Heidelberg) und 0,03% Na-Stearat enthält (PBS-T-TS)) für zwei Stunden bei Raumtemperatur inkubiert. Nach Entfernen der Lösungen werden alle Vertiefungen 5–6mal mit je 300 µl PSB-0,5% Tween® 20 gewaschen (Standzeit bei jedem Waschschritt je 5 min.). Anschließend wird je 150 µl einer 1:1000 Verdünnung von affinitätschromatographisch gereinigten Alkalisch-Phosphatase-konjugierten F(ab')$_2$-Fragmenten von Ziegen-anti-human-IgG (Fc) Antikörpern in PBS-T-ST beschickt und 2 Stunden bei Raumtemperatur inkubiert. Nach nochmaligem 5–6mal Waschen mit PBS-0,5% Tween® erfolgt die Zugabe von je 150 µl der Substratlösung (1 mg/ml p-Nitrophenylphosphat in Diäthanolaminpuffer, pH 9,0) und eine Inkubation für 30 min. bei Raumtemperatur. Die Reaktion wird durch Zugabe von je 50 µl einer 0,4 M Lösung von EDTA (Titriplex 111, Merck, Darmstadt) beendet und die Enzymreaktion kann bei 406 nm etwa in einem DYNATECH Mikroplat-Reader MR 600 gegen eine Referenzwellenlänge von 496 nm abgelesen werden.

Negative Seren erbringen Extinktionswerte von etwa 0,040–0,060. Hochpositive Seren, die etwa bei dem Amersham Anti-DNS Kit 300 Units zeigen (Normalwert 0–25 Units), zeigen Extinktionswerte >2000. Die Grenze zwischen negativen und positiven Seren liegt bei einer Extinktion von 0,100 (Mittelwert plus 2 SD von 120 Seren normaler Blutspender).

Die gebundene DNS wird durch die Carbodiimidreaktion nicht denaturiert, da

(1) eine Behandlung der mit DNS beschichteten Platten mit Nuclease S1 (Böhringer Mannheim, Best. Nr. 818330) nicht zu einer Minderung der Extinktion mit Seren führt, die Anti-Doppelstrang-DNS Antikörper enthielt und da

(2) die Bindung dieser Antikörper durch ihre Vorinkubation mit PM2-Phagen-DNS nach deren Nuclease S1 (Böhringer Mannheim, Best. Nr. 818330) Behandlung (um nur Doppelstrang-DNS zu erhalten) bis auf die Werte von Negativseren unterdrückt werden konnte.

**Patentansprüche**

1. Verfahren zum Immobilisieren von Substanzen an Kunststoffestphasen, die mit Weichmachern versetzt sind, dadurch gekennzeichnet, daß geeignete aktivierbare Gruppen in den Weichmachern oder Additiven verwendet werden, um die Substanzen an die Kunststoffestphasen zu binden oder zu diesem Zweck aktiviert oder mit bi- oder multivalenten Reaktanten umgesetzt werden.

2. Verfahren nach Anspruch 1, dadurch gekennzeichnet, daß die Aktivierung durch Behandlung der Kunststoffestphasen mit Carbodiimid-Derivaten erfolgt.

3. Verfahren nach Anspruch 1, dadurch gekennzeichnet, daß die Umsetzung mit Diglutaraldehyd erfolgt.

4. Verfahren nach Anspruch 1, dadurch gekennzeichnet, daß als Weichmacher oder Additive hydrophobe Substanzen mit verwendbaren Gruppen, insbesondere Succinimidylgruppen, Glyoxalgruppen, 2-Pyridyldithio-Gruppen, 4-Fluoro-3-Nitrophenylsulfon-Gruppen oder Imidoester für eine direkte Aktivierung verwendet werden.

5. Verfahren nach Anspruch 1 und/oder Anspruch 2, dadurch gekennzeichnet, daß als Weichmacher oder Additive hydrophobe Substanzen mit aktivierbaren Gruppen, insbesondere Carboxygruppen, –SH-Gruppen, Azidogruppen, oder Phenazylbromide oder Phthalimide verwendet werden.

6. Verfahren nach Anspruch 3, dadurch gekennzeichnet, daß als Additive Stearate, vorzugsweise Zn- oder Na-Stearate verwendet werden.

7. Verfahren nach mindestens einem der vorhergehenden Ansprüche, dadurch gekennzeichnet, daß Antikörper, Antigene, Haptene, Enzyme, DNS, RNS, Lectine, Polysaccharide, Rezeptoren oder Liganden an die aktivierte Kunststoffestphase immobilisiert werden.

8. Verfahren nach mindestens einem der vorhergehenden Ansprüche, dadurch gekennzeichnet, daß als Kunststoffestphasen Polystyrol oder Polyvinylchlorid verwendet werden.

9. Verfahren nach Anspruch 7, dadurch gekennzeichnet, daß die Kunststoffestphasen als Mikrotiterplatten ausgebildet sind.

10. Verfahren nach mindestens einem der vorhergehenden Ansprüche, dadurch gekennzeichnet, daß die Kunststoffestphasen nach der Aktivierung zusätzlich mit den jeweils als Weichmacher oder Additive verwendeten Substanzen nachbehandelt werden.

11. Mit Additiven oder Weichmachern behandelte Kunststoffestphasen, dadurch gekennzeichnet, daß sie nach einem Verfahren gemäß einem der Ansprüche 1 bis 10 hergestellt wurden und in Immunosorbentassays verwendbar sind.

12. Kunststoffestphase nach Anspruch 11, dadurch gekennzeichnet, daß sie in einem enzymabhängigen Immunosorbentassays (ELISA) verwendbar ist.

13. Kunststoffestphase nach Anspruch 11, dadurch gekennzeichnet, daß sie in einem Radioimmunoassay (RIA) verwendbar ist.

**Claims**

1. Process for immobilizing substances on plastic solid phases mixed with plasticizers, characterized in that suitable activatable groups are used in the plasticizers or additives, in order to bind the substances to the plastic solid phases or for this purpose are activated or reacted with divalent or multivalent reactants.

2. Process according to claim 1, characterized in that activation takes place by treatment of the plastic solid phases with carbodiimide derivatives.

3. Process according to claim 1, characterized in that reaction takes place with diglutaraldehyde.

4. Process according to claim 1, characterized in that as plasticizers or additives use is made of hydrophobic substances with usable groups, particularly succinimidyl groups, glyoxyl groups, 2-pyridyldithio groups, 4-fluoro-3-nitrophenylsulphone groups or imidoesters for a direct activation.

5. Process according to claim 1 and/or 2, characterized in that the plasticizers or additives are constituted by hydrophobic substances with activatable groups, particularly carboxy groups, –SH-groups, azido groups or phenazylbromides or phthalimides.

6. Process according to claim 3, characterized in that the additives are constituted by stearates, preferably Zn or Na-stearates.

7. Process according to at least one of the preceding claims, characterized in that antibodies, antigens, haptens, enzymes, DNS, RNS, lectins, polysaccharides, receptors or ligands are immobilized on the activated plastic solid phase.

8. Process according to at least one of the preceding claims, characterized in that polystyrene or polyvinylchloride are used as the plastic solid phases.

9. Process according to claim 7, characterized in that the plastic solid phases are in the form of microtitre plates.

10. Process according to at least one of the preceding claims, characterized in that, following activation, the plastic solid phases are additionally subsequently treated with the substances used as the plasticizer or additive.

11. Plastic solid phases treated with additives or plasticizers, characterized in that they are prepared according to a process according to one of claims 1 to 10 and are usable in immunosorbent assays.

12. Plastic solid phase according to claim 11, characterized in that it is usable in an enzyme-dependent immunosorbent assay (ELISA).

13. Plastic solid phase according to claim 11, characterized in that it is usable in a radioimmuno assay (RIA).

**Revendications**

1. Procédé pour l'immobilisation de substances sur des phases solides en matière plastique qui sont mélangées à des plastifiants, caractérisé par le fait que des radicaux appropriés qui peuvent être activés sont utilisés dans les plastifiants ou les additifs pour lier les substances aux phases solides en matière plastique, ou sont activés à cette fin, ou peuvent être amenés à réagir avec des réactifs bivalents ou polyvalents.

2. Procédé selon la revendication 1, caractérisé par le fait que l'activation a lieu par traitement des phases solides en matière plastique avec des dérivés de carbodiimide.

3. Procédé selon la revendication 1, caractérisé par le fait que la réaction a lieu avec de l'aldéhyde diglutarique.

4. Procédé selon la revendication 1, caractérisé par le fait que l'on utilise comme plastifiants ou comme additifs des substances hydrophobes comprenant des radicaux utilisables, en particulier des radicaux succinimidoyles, des radicaux glyoxaliques, des radicaux pyridyl-2 dithio, des radicaux fluoro-4 nitro-3 phénylsulfoniques, ou un imido-éther pour une activation directe.

5. Procédé selon la revendication 1 et/ou la revendication 2, caractérisé par le fait que l'on utilise comme plastifiants ou comme additifs des substances hydrophobes comprenant des radicaux qui peuvent être activés, en particulier des radicaux carboxyliques, des radicaux −SH, des radicaux azido, ou la bromophénazyle, ou la phtalimide.

6. Procédé selon la revendication 3, caractérisé par le fait que l'on utilise comme additifs des stéarates, de préférence les stéarates de Zn ou de Na.

7. Procédé selon l'une au moins des revendications précédentes, caractérisé par le fait que l'on immobilise sur la phase solide activée en matière plastique des anticorps, des antigènes, des haptènes, des enzymes, de l'ADN, de l'ARN, de la lectine, des polysaccharides, des récepteurs ou des ligands.

8. Procédé selon l'une au moins des revendications précédentes, caractérisé par le fait que l'on utilise comme phase solide en matière plastique du polystyrène ou du chlorure de polyvinyle.

9. Procédé selon la revendication 7, caractérisé par le fait que les phases solides en matière plastique sont constituées par des microplaquettes de dosage.

10. Procédé selon l'une des revendications précédentes, caractérisé par le fait que les phases solides en matière plastique, après leur activation, sont soumises en outre à un traitement ultérieur par les substances qui sont utilisées à chaque fois comme plastifiants ou comme additifs.

11. Phases solides en matière plastique traitées par des additifs ou des plastifiants, caractérisées par le fait qu'elles ont été fabriquées par un procédé selon l'une des revendications 1 à 10, et qu'elles peuvent être utilisées pour des immunoessais.

12. Phase solide en matière plastique selon la revendication 11, caractérisée par le fait qu'elle peut être utilisée pour un essai immunoenzymatique (Enzyme-linked Immunosorbentassay ou ELISA).

13. Phase solide en matière plastique selon la revendication 1, caractérisée par le fait qu'elle peut être utilisée pour un essai radioimmunologique (Radioimmunoassay ou RIA).